# EUROPEAN PATENT SPECIFICATION

(11) **EP 0 381 514 B1**
(45) Date of publication and mention of the grant of the patent: **17.05.1995**
(21) Application number: 90301099.9
(22) Date of filing: 02.02.1990
(51) Int. Cl.: A61K 31/13

(54) **Use of N-methyl-sphingosine as inhibitor of cell growth**
Verwendung von N-Methyl-Sphingosin als Zell-Wachstums-Inhibitor
Utilisation de la N-méthyl-sphingosine comme inhibiteur de la croissance cellulaire

(30) Priority: 03.02.1989 US 306378; 07.08.1989 US 390135
(43) Date of publication of application: 08.08.1990
(73) Proprietor: The Biomembrane Institute, Seattle Washington 98119 (US); CORNELL RESEARCH FOUNDATION, INC., Ithaca New York 14850 (US); MECT CORPORATION, Tokyo 163 (JP)
(72) Inventor: Igarashi, Yasuyuki, Bellevue, Washington 98004 (US); Toyokuni, Tatsushi, West Seatttle, Washington 98119 (US); Hakomori, Sen-itiroh, Mercer Island, Washington 98040 (US); Racker, Efraim, Department of Biochemistry, Ithaca, New York 14853 (US); Fujita, Shuji, Tachikawashi, Tokyo (JP); Sugimoto, Mamoru, Nakano-ku, Tokyo (JP); Ito, Masayoshi, Kunitachi-shi, Tokyo (JP); Shitori, Yoshiyasu, Shinjuku-ku, Tokyo (JP); Ogawa, Tomoya, Musashino-shi, Tokyo (JP)
(74) Representative: Woodcraft, David Charles

(56) References cited:
- WO-A-88/01869
- SCIENCE, 1990, vol. 248, p. 1653-1656, Ghosh et al
- Biochemistry, 1989, vol. 28, p. 3138-3145, Merrill et al

## Description

This invention relates to inhibitors of cell growth due in part to inhibition of some protein kinases and also due to other as yet unexplored mechanisms, and in particular this invention relates to the recent findings by the present inventors that N-methyl-sphingosine is an especially strong inhibitor of growth of many cells including tumor cells, cells involved in immune responses and cells involved in inflammatory responses. This finding opens the possibility of using these compounds as effective growth inhibitors of cells such as those mentioned above. Unless otherwise specified, "sphingosine" means sphingosine irrespective of D- or L- or erythro- or threo-configuration. Unless otherwise specified "N-methyl-sphingosine" means N,N-dimethyl-sphingosine or N-monomethyl-sphingosine, irrespective of D- or L- or erythro- or threo-configuration.

Sphingosine and sphingoid base have been implicated as inhibitors of C-kinase and EGF receptor-associated tyrosine kinase (Hannun and Bell, Science, 235, 670-674, 1987; Hannun, et al JBC, 261, 12604-12609, 1986; Kreutter, et al, JBC, 262, 1632-1637, 1987). In these studies however, the sphingosine used was from a commercial source (Sigma Chemical Company). The preparation contained various impurities, such as 3-0, 5-0 methyl sphingosine and N-methyl sphingosine, since commercially available sphingosine is prepared after methanolysis of sphingomyelin or cerebroside, and these impurities are introduced during methanolysis. Furthermore, the backbone structure of sphingosine, i.e., the D-erythro configuration, is partially converted to the D-threo configuration. Therefore, based on these changes and various structures present in commercial sphingosine, the observed claim that sphingosine is an important inhibitor for C-kinase as well as EGF-receptor kinase remains ambiguous.

However, because of the potential growth modifying activities of sphingosine and sphingoid base and thus possible application of these compounds to modulate cell growth, this area remains an exciting area for investigation.

WO88/01869 describes the inhibition of protein kinase C by sphingosine and analogs thereof. Structure/activity tests lead to the conclusion that a long alkyl chain and a free amino group were essential for activity.

It has now been unexpectedly found that a free amino group is not essential for inhibition of protein kinase activity.

According to the invention there is provided

A medicament comprising:
i one or more cell growth inhibitors selected from synthetically prepared N-methyl sphingosine, N-methyl sphingosine prepared from naturally occuring sphingolipid and pharmaceutically acceptable salts thereof, and
ii a pharmaceutically acceptable carrier diluent or excipient.

According to the invention there is provided One or more cell growth inhibitors selected from synthetically prepared synthetically prepared N-methyl sphingosine, N-methyl-sphingosine prepared from naturally occurring sphingolipid and pharmaceutically acceptable salts for use as a medicament.

According to the invention there is provided the use of one or more cell growth inhibitor selected from synthetically prepared N-methyl sphingosine, N-methyl-sphingosine prepared from naturally occurring sphingolipid and pharmaceutically acceptable salts thereof in the manufacture of a medicament for inhibiting growth of human, mammalian or other animal tumors.

According to the inventiion there is provided the use of one or more cell growth inhibitors selected from synthetically prepared N-methyl sphingosine, N-methyl-sphingosine prepared from naturally occurring sphingolipid and pharmaceutically acceptable salts thereof in the manufacture of a medicament for inhibiting metastasis of human, mammalian or other animal tumors.

According to the invention there is provided the use of one or more cell growth inhibitors selected from synthetically prepared N-methyl sphingosine, N-methyl-sphingosine prepared from naturally occurring sphingolipid and pharmaceutically acceptable salts thereof in the manufacture of a medicament for inhibiting human and animal, preferably mammalian, immune responses due at least in part to lymphocyte mitogenesis.

According to the invention there is provided the use of one or more cell growth inhibitors selected from synthetically prepared N-methyl sphingosine, N-methyl-sphingosine prepared from naturally occurring sphingolipid and pharmaceutically acceptable salts thereof for the manufacture of a medicament for inhibiting human and animal inflammatory response due at least in part to granulocyte or lymphocyte mitogenesis.

According to the invention there is provided a method of inhibiting growth of human and animal cells other than a method for treatment of the human or animal body by surgery therapy or diagnosis performed on the human or animal body comprising contacting the cells with one or more cell growth inhibitors selected from synthetically prepared N-methyl sphingosine, N-methyl-sphingosine prepared from naturally occurring sphingolipid and pharmaceutically acceptable salts thereof.

In preferred embodiments, the growth inhibitor comprises synthetically prepared N,N-dimethyl-D-erythro-sphingenine, synthetically prepared N,N-dimethyl-L-erythro-sphingenine or pharmaceutically acceptable salts thereof. Especially preferred is synthetically prepared N,N-dimethyl-D-erythro-sphingenine or pharmaceutically acceptable salts thereof.

### BRIEF DESCRIPTION OF THE DRAWINGS

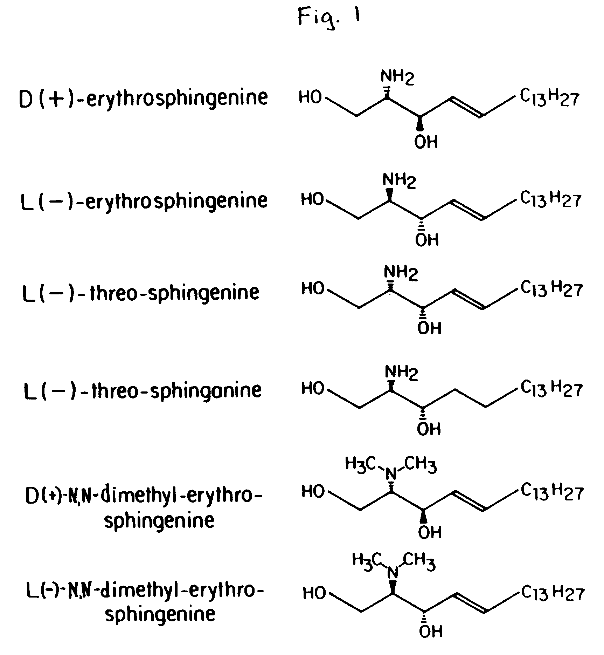

Fig. 1 shows the structures of sphingosine and various synthetically prepared sphingosine derivatives.

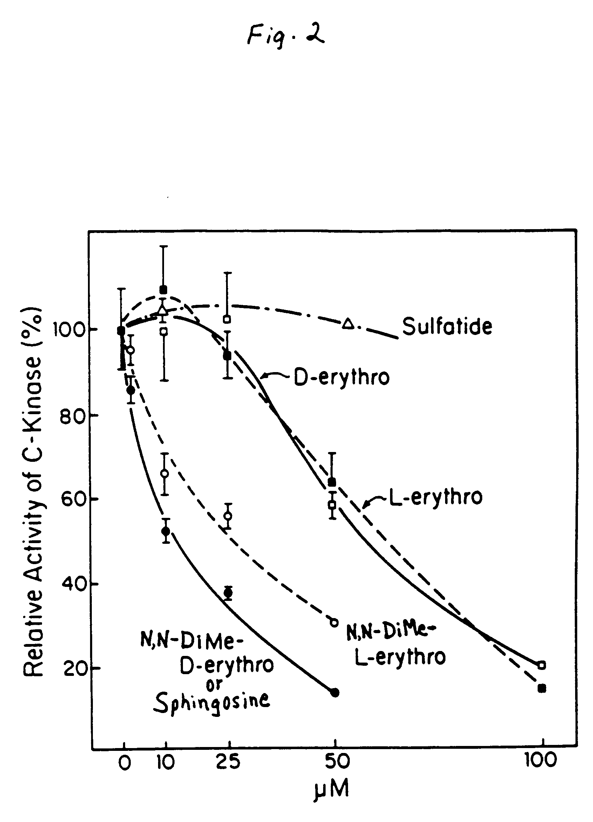

Fig. 2 is a graph showing the inhibitory effect of sphingosine and various synthetically prepared sphingosine derivatives on the activity of C-kinase. The abscissa represents the concentration of sphingosine, synthetically prepared sphingosine derivatives, sphingosine prepared from naturally occuring sphingolipid or sulfatide in »M. The ordinate represents the relative activity of C-kinase (%). Closed circles: synthetically prepared N,N-dimethyl-D-erythro-sphingenine (N,N-DiMe-D-erythro) or sphingosine prepared from naturally occurring sphingolipid (sphingosine); Open circles: N,N-Dimethyl-L-erythro-sphingenine (N,N-DiMe-L-erythro); Closed squares: L-erythro sphingenine (L-erythro); Open squares: D-erythro-sphingenine (D-erythro); Open triangles: Sulfatide.

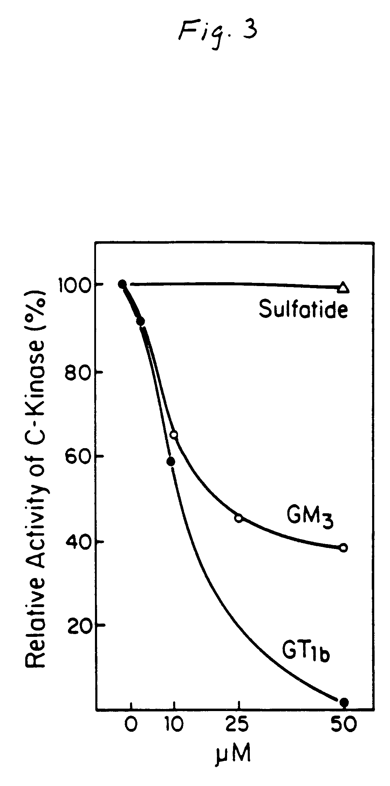

Fig. 3 is a graph showing the inhibitory effect of gangliosides and sulfatide on the activity of C-kinase. The abscissa represents the concentration of the gangliosides or of sulfatide in »M. The ordinate represents the relative activity of C-kinase (%). Closed circles: GT_{1b}; Open circles: GM₃; Open triangles: sulfatide. The glycolipids are abbreviated according to the system of Svennerholm (Svennerholm, L. J. Neurochem, 10, 613-623, 1963).

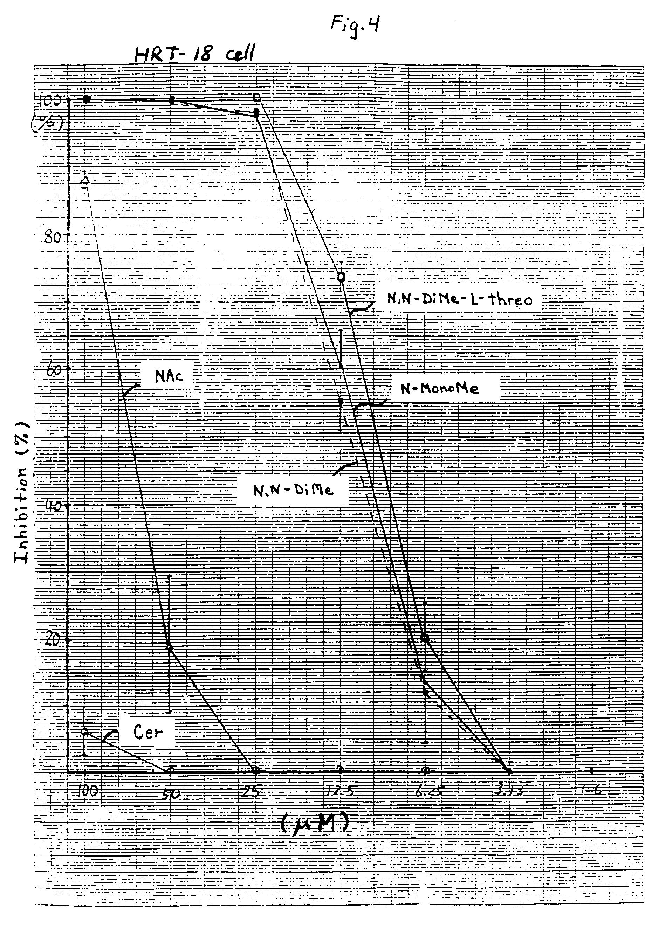

Fig. 4 is a graph showing the effect of N-methyl-sphingosine prepared from naturally occuring sphingolipid on growth of human colonic cancer cells HRT-18. The abscissa represents concentration of sphingosine derivatives or ceramide in »M. The ordinate represents the relative inhibition of cell growth (%). Open circles: ceramide (cer); Open triangle: N-acetylsphingosine (NAc); Closed squares: N,N-dimethyl-sphingosine (N,N-DiMe) prepared from naturally occuring sphingolipid; Closed circles; N-monomethyl-sphingosine (N-MonoMe) prepared from naturally occuring sphingolipid; Open squares: N,N-dimethyl-L-threo-sphingosine (N,N-DiMe-L-threo) prepared from naturally occuring sphingolipid.

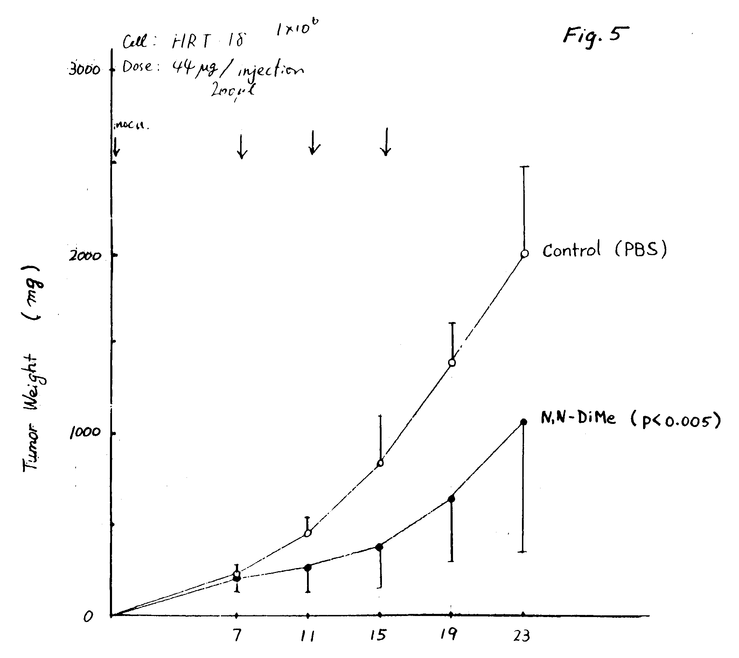

Fig. 5 is a graph showing inhibition of human cancer cell growth in nude mice by N,N-dimethyl-sphingosine (N,N-DiMe) prepared from naturally occuring sphingolipid. The abscissa represents days. The ordinate represents tumor weight (mg). The control was phosphate buffered saline (PBS).

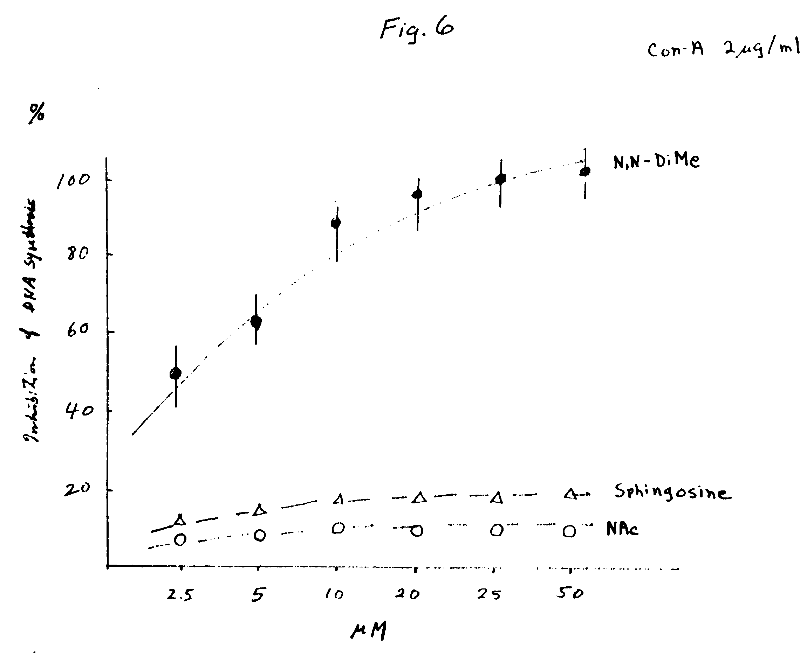

Fig. 6 is a graph showing the inhibition of concanavalin-A-induced mitogenesis of human peripheral blood lymphocytes by N,N-dimethyl-sphingosine prepared from naturally occuring sphingolipid. The abscissa represents the concentration, in »M, of N,N-dimethyl-sphingosine (N,N-DiMe: closed circles) prepared from naturally occuring sphingolipid, sphingosine (open triangles) or N-acetylsphingosine (NAc: open circles). The ordinate represents relative inhibition of DNA synthesis (%).

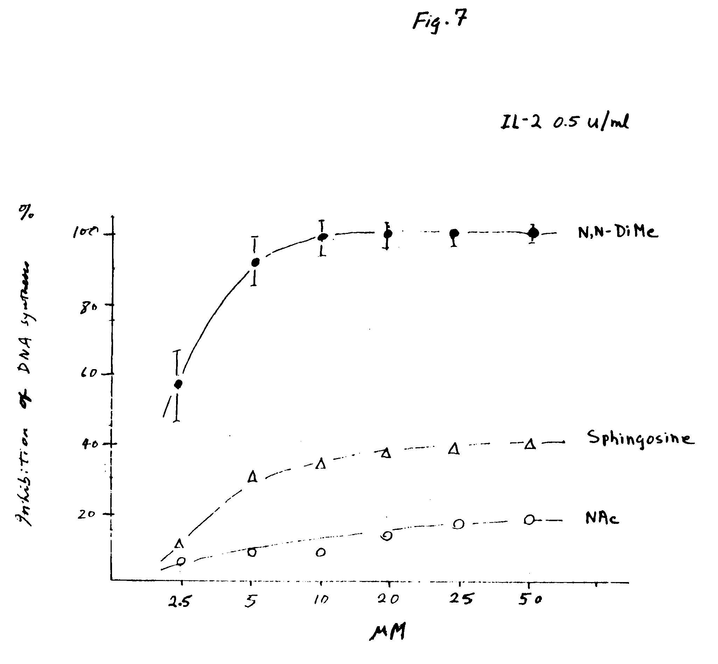

Fig. 7 is a graph showing the inhibition of IL-2-induced mitogenesis of human peripheral blood lymphocytes by N,N-dimethyl-sphingosine prepared from naturally occuring sphingolipid. The abscissa represents the concentration, in »M, of N,N-dimethyl-sphingosine (N,N-DiMe: closed circles) prepared from naturally occuring sphingolipid, sphingosine (open triangles) or N-acetylsphingosine (NAc: open circles). The ordinate represents relative inhibition of DNA synthesis (%).

As used herein, sphingosine means sphingosine irrespective of D- or L- or erythro- or threo-configuration.

As used herein, N-methyl-sphingosine means N,N-dimethyl-sphingosine or N-monomethyl-sphingosine irrespective of D- or L-/erythro or threo configuration, unless otherwise specified.

Also, as used herein, "inhibiting" means "preventing and/or slowing".

Further, as used herein, "synthetically prepared" N-methyl-sphingosine means N-methyl-sphingosine prepared from a synthetically prepared sphingosine backbone, i.e. the structure is a synthetic product, and N-methyl-sphingosine "prepared from naturally occuring sphingolipid" means N-methyl-sphingosine prepared from sphingolipids which occur naturally, such as, for example, sphingomyelin, cerebroside, etc.

In order to clarify the ambiguity regarding whether sphingosine is an important inhibitor of protein kinases, including C-kinase and EGF-receptor kinase, and thus is a potential candidate for inhibiting cell growth, the present inventors used synthetically prepared chemically well-defined D- and L-erythro- and threo- sphingosine and their N,N-dimethyl derivatives as well as sphingosine prepared from naturally occurring sphingolipid as shown in Fig. 1 and evaluated the protein kinase inhibitory activity of these compounds. Comparison of the effect of chemically well-defined synthetically prepared stereo isomers of sphingosine and their derivatives on C-kinase activity from A413 cells (human epidermol carcinoma cells) is shown in Fig. 2, Fig. 3, and Tables I and II (See Example 2).

The following conclusions can be drawn:
1. D-erythro and L-erythro sphingenine showed a weak and similar inhibitory activity.
2. Synthetically prepared N,N-dimethyl-D-erythro-sphingenine showed a strikingly strong inhibitory activity of C-kinase. Synthetically prepared N,N-dimethyl-L-erythro-sphingenine showed a weaker inhibitory activity, which was still stronger than non-substituted sphingenine having free amino groups.
3. The inhibitory activity of synthetically prepared N,N-dimethyl-D-erythro-sphingenine was higher than N-acetyl-GM₃, N-glycolyl-GM₃, and GD₁ₐ gangliosides but had a similar range of inhibitory activity as the most potent inhibitory ganglioside GT_{1b}.
4. By analogy, synthetically prepared N,N-dimethyl-D-erythro-sphingenine and synthetically prepared N,N-dimethyl-L-erythro-sphingenine are expected to show similar activity on EGF receptor kinase.

Synthetically prepared N-methyl-sphingosines, including, N,N-dimethyl-D-erythro-sphingenine and N-methyl-L-erythro-sphingenine can be prepared by known methods comprising reductive methylation of the corresponding unmethylated sphingosine, for example D(+)erythro-sphingenine and L(-)erythro-sphingenine, respectively, in the presence of formaldehyde and sodium borohydride (Means and Feeney, Biochemistry, 7, 2192, 1968; Chemical Modification of Proteins, Holden-Day, Inc., San Francisco, p. 217, 1971). The resulting synthetically prepared N,N-dimethyl-sphingosine or sphingenine derivatives can be purified by TLC or HPLC. N-monomethyl derivatives were synthesized from backbone sphingenine by successive N-tert-butyloxycarbonylation (di-ter-butyldicarbonate-NaHCO₃), O-acetylation (acetic anhydride-pyridine), N-methylation (methyl iodide-sodium hydride-dimethyl-formamide), and deprotection. The resulting compound was also purified by TLC and HPLC.

The synthetically prepared backbone sphingenines, D(+)erythro-sphingenine and L(-)erythro-sphingenine, can be prepared by various known methods, either starting from L-serine (Radunz H-E, Devant RM, Eiermann V, Liebigs Ann Chem 1103-1105, 1988; Nimkar S, Menaldino D, Merrill AH, Liotta D, Tetrahedron Lett, 29: 3037-3040, 1988; Herold P, Helvetica Chimica Acta 71: 354-362, 1988), or starting from D-glucose (Koike, K., et al Carbohydrate Research, 158, 113-123, 1986; Koike, K., et al, An efficient synthesis of ceramide from D-Glucose, Glycoconjugate Journal, 1, 107-109, 1984). The structure of sphingosine and of the N-methyl derivatives can be confirmed by NMR spectrometry and fast atom bombardment mass spectrometry.

In addition to the above, some of the present inventors also discovered that N-methyl-sphingosine prepared from naturally occuring sphingolipid has qualitatively analogous action to synthetically prepared N-methyl-sphingosine.

The sphingosine backbone can be prepared by two known methods: (1) methanolysis of natrual sphingolipid according to Tielfelder, H. and Klenk, E. (Die Chemie der Cerebrocide and Phosphatide, J. Springer, Berlin, 1930) and modified by Gaver, R.C. and Sweeley, C.C. (J. Am. Oils Chem. Soc., 42: 264-298 1965); and (2) enzymatic hydrolysis of natural sphingolipids by glycosidase and ceramides as described by Kanfer in Hakomori, S., Handbook of Lipid Research, Volume 3, Sphingolipid Biochemistry, Kafer, J.N. and Hakamori, S. (Eds.), Plenum, New York, pages 167-247 (1983).

N-methyl-sphingosine can be prepared from naturally occuring sphingolipid by N-methylation in the presence of formaldehyde and sodium borohydride (Means, G.E. & Feeney, R.E., Biochemistry, 7, 2192-2201, 1968).

Suitable sources of naturally occurring sphingolipid include brain, kidney, spleen, blood cells and other organs and tissues from animals. Yeast and plant grains are also expected to be suitable sources of naturally occurring sphingolipid.

The structure of sphingosine or N-methyl-sphingosine can be confirmed by mass spectrometry and ¹H-NMR spectroscopy.

Some of the present inventors compared the effect of N-methyl-sphingosine prepared from naturally occuring sphingolipid to unmethylated sphingosine. The comparisons are shown in Figs. 4 to 7. From these data, the following conclusions can be drawn.
1. N,N-dimethyl-sphingosine, N-monomethyl-sphingosine and N,N-dimethyl-L-threo-sphingosine prepared from naturally occuring sphingolipid showed strong inhibitory activity on growth of human colonic cancer cells in vitro.
2. N,N-dimethyl-sphingosine prepared from naturally occuring sphingolipid showed a statistically significant inhibitory effect on in vivo growth of human tumor cells in nude mice.
3. N,N-dimethyl-sphingosine prepared from naturally occuring sphingolipid showed a strong inhibitory effect on concanavalin-A-induced mitogenesis of human peripheral blood lymphocytes and of mouse splenocytes.
4. N,N-dimethyl-sphingosine prepared from naturally occuring sphingolipid showed a strong inhibitory effect on IL-2-dependent mitogenesis of human peripheral blood lymphocytes and of mouse splenocytes.

For inhibiting growth of tumor cells, tumors and metastasis of tumors the medicaments and methods of the present invention are expected to be especially effective when used in combination with existing chemotherapeutic agents, such as, for example, mitomycin and 5-fluorouracil.

Suitable pharmaceutically acceptable salts of synthetically prepared sphingosine, synthetically prepared N-methyl-sphingosine, sphingosine prepared from naturally occurring sphingolipid and N-methyl-sphingosine prepared from naturally occuring sphingolipid can readily be determined by the skilled artisan.

Suitable pharmaceutically acceptable carriers, diluents or excipients for the medicaments of the present invention depend upon the particular medical use of the medicament and can readily be determined by the skilled artisan.

Suitable methods of administration of the medicaments of the present invention depend upon the particular medical application and can readily be determined by the skilled artisan.

Suitable doses of the medicaments of the present invention depend upon the particular medical application, as well as the weight of the subject, etc., and can readily be determined by the skilled artisan for example by extrapolation from in vitro data such as that shown in Fig. 2 and Table 1 for sphingosine prepared from naturally occurring sphingolipid and synthetically prepared N-methyl-sphingosine and such as that shown in Fig. 4 for N-methyl-sphingosine prepared from naturally occuring sphingolipid or from in vivo data such as that shown in Fig. 5 for N-methyl-sphingosine prepared from naturally occuring sphingolipid.

The present invention will now be described by reference to specific examples.

Unless otherwise specified, all percents, ratios, etc., are by weight.

### EXAMPLE I

### SYNTHESIS OF SYNTHETICALLY PREPARED SPHINGOSINE DERIVATIVES

D(+)-erythro-sphingenine, L(-)-erythro-sphingenine, L(-)-threo-sphingenine, and L(-)-threo-sphingenine were synthesized as previously described (Koike, K., et al Carbohydrate Research, 158, 113-123, 1986; Koike, K., et al, An efficient synthesis of ceramide from D-Glucose, Glycoconjugate Journal, 1, 107-109, 1984). N,N-dimethyl derivatives were prepared by reductive methylation of erythro-sphingenine in the presence of formaldehyde and sodium borohydride (Means & Feeney, Biochemistry, 7, 2192, 1968; Chemical Modification of Proteins, Holden-Day, Inc., San Francisco, p. 217, 1971).

The synthetically prepared backbone sphingenines, D(+)erythro-sphingene and L(-)erythro-sphingenine, can also be prepared by various known methods, either starting from L-serine (Radunz H-E, Devant RM, Eiermann V, Liebigs Ann Chem 1103-1105, 1988; Nimkar S, Senaldino D, Merrill AH, Liotta D, Tetrahedron Lett, 29: 3037-3040, 1988; Herold P, Helvetica Chimica Acta 71: 354-362, 1988), or starting from D-glucose. (Koike et al, supra).

For comparison, crude sphingosine was prepared from cerebroside by methanolysis as described originally by Tierfelder and Klenk (Die Chemie de Cerebroside und Phosphatide, Springer Verlag, Berlin, 1932).

The structures of the N,N-dimethyl-sphingosine derivatives were confirmed by NMR spectroscopy and fast atom bombardment mass spectrometry.

### EXAMPLE 2

### DETERMINATION OF INHIBITION OF C-KINASE ACTIVITY BY SPHINGOSINES AND SYNTHETICALLY PREPARED SPHINGOSINE DERIVATIVES

### Isolation of C-kinase from A431 cells

A431 cells were grown in a mixture of DME and Ham's F-12 medium (weight ratio 1:1) supplemented with 10% fetal calf serum (FCS). Cells harvested from 50 150-cm diameter dishes were treated simultaneously for partial purification of C-kinase, by the method of Kreutter et al. (JBC, 262, 1633-1637, 1987). Briefly, cells were scraped by rubber policeman, suspended in 50 ml of 20 mM Tris-HCL (pH 7.5), 2 mM EDTA, 0.5 mM EGTA, 0.15 U/ml aprotinine, and 0.25 M sucrose, and homogenized by 50 strokes at 4°C in a Dounce homogenizer (40-ml size, Wheaton). The homogenized cells were ultracentrifuged at 100,000 xg for 60 min. The supernatant was purified on a DE52 column equilibrated with 20 mM Tris-HCL (pH 7.5), 2 mM EDTA, and 0.5 mM EGTA (buffer B), and washed well with this buffer. The C-kinase activity was eluted with buffer B containing 0.1 M NaCl. The activity in this fraction was 200-500 pmol P/min/mg protein. The fraction, which was free of A-kinase and other kinases, was aliquoted and kept at -80°C.

### Determination of Inhibitory Effects of Sphingosine and Sphingosine Derivatives on C-kinase

In view of the extremely variable results obtained in a mixed micelle system, as described by Bell and Hannun (Science 235, 670-674, 1987), the standard liposome method described by Kreutter et al. (JBC, 260 5979-5986, 1985) was slightly modified, and the effect of sphingosine and sphingosine derivatives (non-glycosylated and glycosylated) was studied under these conditions. In conical tubes (1.5 ml content, Sarstedt), phosphatidylserine (5 »g/tube) and 1,2-diolein (0.05 »g/tube), with or without appropriate quantity of sphingosine (sphingosine prepared from naturally occurring sphingolipid was prepared as described in Example 3), its derivatives or sulfatide, were added in organic solvent (ethanol or chloroform-methanol), and the mixture was evaporated under an N₂ stream. The lipid mixture was sonicated in 30 »l of 20 mM Tris-HCl (pH 7.5) for 30 min. The liposomes in the tube were supplemented with the reaction mixture, consisting of 25 mM Tris-HCl (pH 7.5), 10 mM MgCl₂, 400 »M EDTA, 50 »M EGTA, 500 »M CaCl₂, 200 »g/ml Histone III-S, and 20 »M gamma-[³²P]-ATP (2 x 10⁶ cpm); final volume was 90 »l.

The reaction was initiated by addition of 10 »l of C-kinase fraction (containing 1-2 »g protein) prepared as described above, and the reaction mixture was incubated for 10 min at 30°C. The reaction was terminated by addition of 1 ml of 25% TCA with 200 »l of 1% BSA in 1 mM ATP solution (pH 7.5). The precipitate was centrifuged, washed twice with 1 ml of 25% TCA, dissolved in 1 ml of 1 N NaOH containing 0.1% deoxycholate with slight heating (80°C for 10 min), and counted in a scintillation counter. The value without phosphatidylserine, 1,2-diolein, or Ca²⁺ were used as a control. The effects of sphingosine and sphingosine derivatives and are shown in Fig. 2 and Fig. 3 and are summarized in Table I and Table II below.

**TABLE I**

| Inhibitory effect of sphingosine and various sphingosine derivatives on C-kinase activity | |
|---|---|
| Added sphingolipid | Inhibition of C-kinase (concentration in »M yielding 50% inhibition) |
| A. Sphingosines and non-glycoslyated derivatives | |
| Ceramide | no inhibition |
| Sphingosine (D-erythro, SigmaSM) | 25 |
| Sphingosine (D-erythro, SigmaCer) | 25 |
| Sphingosine (D-erythro) | 55 |
| Sphingosine (L-erythro) | 55 |
| Sphingenine (L-threo) | 30 |
| Sphinganine (L-threo) | 50 |
| Sphingenine (DL-erythro, Sigma) | no data |
| N-acetylsphingosine | no inhibition |
| D-erythro-N,N-dimethyl-sphingosine | 5-10 |
| L-erythro-N,N-dimethyl-sphingosine | 25 |
| Sphingosine prepared from naturally occurring sphingolipid | 5-10 |

| B. Glycosylated derivatives | |
|---|---|
| Lactosylsphingosine | no inhibition |
| GM₃ (N-acetyl) | 20 |
| GM₃ (N-glycolyl) | 25 |
| GD₁ₐ | 20 |
| GT_{1b} | 10 |

**TABLE II**

| Inhibition of C-kinase activity by sphingosines | | | |
|---|---|---|---|
| Lipid Added | Amount (»M) | C-kinase ³²P incorporation | % |
| Phosphatidyl-serine, diolein, Ca⁺² | * | 39620 ± 4156 | 100 ± 10 |
| D-erythro-sphingenine | 50 »M | 18754 ± 2184 | 47 ± 6 |
| L-erythro-sphingenine | 50 »M | 18549 ± 2760 | 47 ± 7 |
| N,N-dimethyl-D-erythro-sphingenine | 2 »M | 34206 ± 1220 | 86 ± 3 |
| | 10 »M | 20563 ± 1173 | 52 ± 3 |
| I₅₀ = 10 »M | 25 »M | 15214 ± 377 | 38 ± 1 |
| | 50 »M | 5254 | 13 |
| N,N-dimethyl-L-erythro-sphingenine | 2 »M | 37354 ± 1420 | 95 ± 4 |
| | 10 »M | 25583 ± 1931 | 65 ± 5 |
| I₅₀ = 25 »M | 25 »M | 21743 ± 577 | 55 ± 2 |
| | 50 »M | 11020 | 28 |
| * See text | | | |

From the data shown in Table I and Table II and the results in Fig. 2 and Fig. 3, the following conclusions can be drawn:
1. Synthetically prepared D-erythro and L-erythro sphingenine showed a weak and similar inhibitory activity.
2. Synthetically prepared N,N-dimethyl-D-erythro-sphingenine showed a strikingly strong inhibitory activity of C-kinase. N,N-dimethyl-L-erythro-sphingenine showed a weaker inhibitory activity, which was still stronger than non-substituted sphingenine having free amino groups.
3. The inhibitory activity of synthetically prepared N,N-dimethyl-D-erythro-sphingenine was higher than N-acetyl-GM₃, N-glycolyl-GM₃, and GD₁ₐ gangliosides but had a similar range of inhibitory activity as the most potent inhibitory ganglioside GT_{1b}.

### EXAMPLE 3

### SYNTHESIS OF SPHINGOSINE DERIVATIVES PREPARED FROM NATURALLY OCCURRING SPHINGOLIPID

Sphingosine was prepared after methanolysis of brain sphingolipid fraction according to methods originally described by Thierfelder H. & Klenk E. (Die Chemie der Cerebroside und Phosphatide, J. Springer, Berlin, 1930) and modified by Gaver R.C. and Sweeley C.C. (J Am Oil Chem Soc 42: 294-298, 1965). Briefly, crude sphingolipid was dissolved in methanol containing 0.1 M HCl (prepared from aqueous concentrated HCl), and heated under reflux overnight. The methanolic HCl solution was shaken out with petroleum ether (hexane) to eliminate fatty acids. The lower phase was neutralized, pH adjusted to 10 with NaOH, followed by shaking out with ethylether. The ethylether layer (upper layer) containing sphingosine base was evaporated to dryness and further purified on HPLC, and the peak corresponding to D-erythrosphigenine was separated
Sphingosine can also be prepared by hydrolysis of sphingolipids with glycosidases and phosphodiesterases followed by treatment with ceramidase (Kanfer J.N., pp. 167-247 in: Kanfer J.N. & Hakomori S. (eds.), Sphingolipid biochemistry (Handbook of Lipid Research, Vol. 3, Plenum, NY, 1983). Briefly, cerebroside can be treated with 3-galactosidase or β-glucosidase in the presence of Triton X-100. Sphingomyelin can be created with phosphodiesterase in the presence of Triton X-100. Thus, the liberated ceramide can then be hydrolyzed with ceramidase to release sphingosine. More recently, a ceramidase which acts directly on sphingolipids has been found in Nocardia sp. (Hirabayashi Y., et al., J Biochem (Tokyo) 103: 1-4, 1988).

N,N-dimethyl and N-monomethyl-sphingosine used for Examples 4 to 7 were prepared exclusively from sphingosine preparations derived from brain sphingolipids followed by N-methylation in the presence of formaldehyde and sodium borohydride (Means, G.E. & Feeney, R.E., Biochemistry, 7, 2192-2201, 1968) and its structure was identified by mass spectrometry and ¹H-NMR spectroscopy.

### EXAMPLE 4

### DETERMINATION OF INHIBITION OF HUMAN COLONIC CANCER CELL GROWTH BY SPHINGOSINES AND SPHINGOSINE DERIVATIVES PREPARED FROM NATURALLY OCCURRING SPHINGOLIPID

The effect of N,N-dimethyl-sphingosine, N-monomethyl-sphingosine and N,N-dimethyl-L-threo-sphingosine (all prepared from naturally occurring sphingoglycolipids as described in Example 3) on growth of human colonic cancer cells HRT-18 (obtained from ATCC) was tested in Dulbecco's modified Eagle's medium as follows.

HRT-18 cells were seeded in plates in Dulbecco's modified Eagle's medium supplemented with 10% fetal calf serum (5 x 10⁵ cells per each 3 cm plate). After the cells were cultured overnight, the medium was replaced with the same media containing various concentrations of N,N-dimethyl- or N-monomethyl sphingosine, N-acetylsphingosine, and ceramide as shown in Fig. 4. Cultures were continued for 3 days, and cell numbers were counted. The percent inhibition of cell numbers in the presence of reagents was calculated as percent of control dishes, which contained no sphingosine derivatives.

The results are shown in Fig. 4.

In Fig. 4 the abscissa represents concentration of sphingosine or ceramide in »M. The ordinate represents the relative inhibition of cell growth (%). Open circles: ceramide (Cer); Open triangles: N-acetylsphingosine (NAc); Closed squares: N,N-Dimethyl-sphingosine (N,N-DiMe) prepared from naturally occurring sphingolipid; Closed circles: N-monomethyl-sphingosine (N-MonoMe) prepared from naturally occurring sphingolipid; Open squares: N,N-dimethyl-L-threo-sphingosine (N,N-DiMe-L-threo) prepared from naturally occurring sphingolipid.

The results show that N,N-dimethyl-sphingosine as well as N-monomethyl-sphingosine and N,N-dimethyl-L-threo-sphingosine significantly inhibited at 12.5 »M concentration, whereas ceramide or N-acetylsphingosine showed minimum inhibition even at 150 »M concentration.

### EXAMPLE 5

### DETERMINATION OF INHIBITION OF HUMAN COLONIC CANCER CELL GROWTH IN NUDE MICE BY SPHINGOSINES AND N,N-DIMETHYLSPHINGOSINE PREPARED FROM NATURALLY OCCURRING SPHINGOLIPID

The effect of N,N-dimethyl-sphingosine (prepared from naturally occurring sphingolipid as described in Example 3) on in vivo growth of HRT18 tumor cells in nude mice was determined as follows.

1 x 10⁶ HRT-18 human colonic cancer cells were inoculated into nude mice at day 0 by known methods. N,N-dimethylsphingosine emulsified in phosphate buffered saline (PBS) (44 »g/200 »l) was injected at day 7, 11, and 15. Tumor growth weight in mg was estimated by the size of the tumors. Each group consisted of ten animals.

The results are shown in Fig. 5.

In Fig. 5, the abscissa represents days and the ordinate represents tumor weight (mg). The open circles represent the control, PBS alone. The closed circles represent N,N-dimethyl-sphingosine (N,N-DiMe).

The results show that in vivo growth of HRT18 tumor cells in nude mice was inhibited by N,N-dimethyl-sphingosine. The inhibition of growth was more than 50%, and statistical treatment showed that the inhibition was significant (p>0.005).

### EXAMPLE 6

### DETERMINATION OF INHIBITION OF CONCANAVALIN-A-INDUCED MITOGENESIS OF HUMAN PERIPHERAL BLOOD LYMPHOCYTES AND MOUSE SPLENOCYTES BY N,N-DIMETHYL-SPHINGOSINE PREPARED FROM NATURALLY OCCURRING SPHINGOLIPID

The effect of N,N-dimethyl-sphingosine (prepared from naturally occurring sphingolipid as described in Example 3) on conconavalin-A (con-A) -induced mitogenesis was tested using human peripheral blood lymphocytes and mouse splenocytes.

Peripheral blood lymphocytes isolated from human blood by known methods were cultured in RPMI containing 2 »g con-A/ml for 18 hours in multiple wells. Subsequently, various concentrations of N,N-dimethylsphingosine, sphingosine, or N-acetylsphingosine as shown in Fig. 6 were added to the culture media and cultured continuously for 5 hours, followed by addition of ³H-thymidine. The thymidine uptake over a 5-hour period was determined. The % inhibition of mitogenesis was calculated based on control value (without addition of any inhibitor).

Inhibition of con-A-induced mitogenisis of mouse splenocytes was determined in an analogous manner.

The results for peripheral blood lymphocytes are shown in Fig. 6.

In Fig. 6, the abscissa represents the concentration, in »M, of N,N-dimethyl-sphingosine (N,N-DiMe: closed circles) prepared from naturally occurring sphingolipid, sphingosine (open triangles) or N-acetylsphingosine (NAc: open circles). The ordinate represents relative inhibition of DNA synthesis (%).

The data in Fig. 6 show that con-A-dependent growth stimulation was greatly inhibited in the presence of N,N-dimethyl-sphingosine but not in the presence of unsubstituted sphingosine or N-acetylsphingosine.

Similar results were obtained using mouse splenocytes.

### EXAMPLE 7

### DETERMINATION OF INHIBITION OF IL-2-DEPENDENT MITOGENESIS OF HUMAN PERIPHERAL BLOOD LYMPHOCYTES AND MOUSE SPLENOCYTES BY N,N-DIMETHYL-SPHINGOSINE PREPARED FROM NATURALLY OCCURRING SPHINGOLIPID

The effect of N,N-dimethyl-sphingosine (prepared from naturally occurring sphingolipid as described in Example 3) on IL-2-dependent mitogenesis was tested using human peripheral blood lymphocytes and mouse splenocytes.

Human peripheral blood lymphocytes isolated from human blood by known methods were cultured in RPMI containing 0.5 U/ml IL-2. Subsequently, the medium was replaced with media containing various concentrations of N,N-dimethylsphingosine, sphingosine, and N-acetylsphingosine as shown in Fig. 7, followed by addition of ³H-thymidine. Mitogenesis was measured by incorporation of ³H-thymidine into DNA over a 5-hour period. Percent inhibition as compared to the control value (without addition of any inhibitor) was determined.

Inhibition of IL-2-induced mitogenesis of mouse splenocytes was determined in an analogous manner.

The results for peripheral blood lymphocytes are shown in Fig. 7.

In Fig. 7, the abscissa represents the concentration, in »M, of N,N-dimethyl-sphingosine (N,N-DiMe: closed circles) prepared from naturally occurring sphingolipid, sphingosine (open triangles) or N-acetylsphingosine (NAc: open circles). The ordinate represents relative inhibition of DNA synthesis (%).

The data in Fig. 7 show that mitogenesis was invariably inhibited in the presence of N,N-dimethyl-sphingosine but less so in the presence of unsubstituted sphingosine or N-acetylsphingosine.

Similar results were obtained using mouse splenocytes.

From the results of Examples 4 to 7, the following conclusions can be drawn:
1. N,N-dimethyl-sphingosine, N-monomethyl-sphingosine and N,N-dimethyl-L-threo-sphingosine prepared from naturally occurring sphingolipid showed strong inhibitory activity on growth of human colonic cancer cells in vitro.
2. N,N-dimethyl-sphingosine prepared from naturally occurring sphingolipid showed a statistically significant inhibitory effect on in vivo growth of human tumor cells in nude mice.
3. N-N-dimethyl-sphingosine prepared from naturally occurring sphingolipid showed a strong inhibitory effect on conconavalin-A-induced mitogenesis of human peripheral blood lymphocytes and of mouse splenocytes.
4. N,N-dimethyl-sphingosine prepared from naturally occurring sphingolipid showed a strong inhibitory effect on IL-2-dependent mitogenesis of human peripheral blood lymphocytes and of mouse splenocytes.

While the invention has been described in detail and with reference to specific embodiments thereof, it will be apparent to one skilled in the art that various changes and modifications can be made therein

## Claims

1. One or more cell growth inhibitors selected from synthetically prepared N-methyl sphingosine, N-methyl-sphingosine prepared from naturally occurring sphingolipid and pharmaceutically acceptable salts for use as a medicament.

2. A cell growth inhibitor as claimed in claim 1 comprising N,N-dimethyl-sphingosine prepared from naturally occurring sphingolipid, or N-monomethyl-sphingosine prepared from naturally occurring sphingolipid or synthetically prepared N,N-dimethyl-D-erythro-sphingenine, synthetically prepared N,N-dimethyl-L-erythro-sphingenine, or pharmaceutically acceptable salts.

3. A cell growth inhibitor as claimed in claim 2 comprising synthetically prepared N,N-dimethyl-D-erythro-sphingenine, or N,N-dimethyl-sphingosine prepared from naturally occurring sphingolipid or pharmaceutically acceptable salts.

4. A cell growth inhibitor as claimed in any one of claims 1 to 3 for use in a medicament in inhibiting mammalian cell growth.

5. A cell growth inhibitor as claimed in any one of claims 1 to 3 for use as a medicament in treating malignant or benign tumor cells.

6. The use of one or more cell growth inhibitors selected from synthetically prepared N-methyl sphingosine N-methyl-sphingosine prepared from naturally occurring sphingolipid and pharmaceutically acceptable salts thereof in the manufacture of a medicament for inhibiting growth of human, mammalian or other animal tumors.

7. The use of one or more cell growth inhibitors selected from synthetically prepared N-methyl sphingosine N-methyl-sphingosine prepared from naturally occurring sphingolipid and pharmaceutically acceptable salts thereof in the manufacture of a medicament for inhibiting metastasis of human, mammalian or other animal tumors.

8. The use of one or more cell growth inhibitors selected from synthetically prepared N-methyl sphingosine, N-methyl-sphingosine prepared from naturally occurring sphingolipid and pharmaceutically acceptable salts thereof in the manufacture of a medicament for inhibiting human and animal, preferably mammalian, immune responses due at least in part to lymphocyte mitogenesis.

9. A use as claimed in claim 8 for the manufacture of a medicament for inhibiting an autoimmune response.

10. A use as claimed in claim 9 for the manufacture of a medicament for inhibiting an autoimmune response due at least in part to IL-2-dependent T-cell growth.

11. A use as claimed in claim 8 or claim 9 for the manufacture of a medicament for inhibiting an immune response due at last in part to conconavalin-A induced mitogenesis of lymphocytes.

12. A use as claimed in claim 8 or claim 9 for the manufacture of a medicament for inhibiting an immune response due at least in part to phytohemagglutinin-A induced mitogenesis of lymphocytes

13. The use of one or more cell growth inhibitors selected from synthetically prepared N-methyl sphingosine, N-methyl-sphingosine prepared from naturally occurring sphingolipid and pharmaceutically acceptable salts thereof for the manufacture of a medicament for inhibiting human and animal inflammatory response due at least in part to granulocyte or lymphocyte mitogenesis.

14. A use as claimed in claim 13 for the manufacture of a medicament for inhibiting mammalian inflammatory response.

15. A use as claimed in claim 13 wherein the inflammatory response is due at least in part to conconavalin-A induced mitogenesis of lymphocytes or phytohemagglutin-A induced mitogenesis of lymphocytes.

16. A use as claimed in any one of claims 6 to 15 wherein the cell growth inhibitors comprise N,N-dimethylsphingosine prepared from naturally occurring sphingolipid, or N-monomethyl-sphingosine prepared from naturally occurring sphingolipid or synthetically prepared N,N-dimethyl-D-erythro-sphingenine, synthetically prepared N,N-dimethyl-L-erythro-sphingenine, or pharmaceutically acceptable salts.

17. A use as claimed in claim 16 wherein the cell growth inhibitor comprises synthetically prepared N,N-dimethyl-D-erythro-sphingenine, or N,N-dimethylsphingosine prepared from naturally occurring sphingolipid or pharmaceutically acceptable salts.

18. A method of inhibiting growth of human and animal cells other than a method for treatment of the human or animal body by surgery therapy or diagnosis performed on the human or animal body comprising contacting the cells with one or more cell growth inhibitors selected from synthetically prepared N-methyl sphingosine, N-methyl-sphingosine prepared from naturally occurring sphingolipid and pharmaceutically acceptable salts thereof.

19. A method as claimed in claim 18 wherein the cell growth inhibitor comprises N,N-dimethyl-sphingosine prepared from naturally occurring sphingolipid, or N-46 monomethyl-sphingosine prepared from naturally occurring sphingolipid or synthetically prepared N,N-dimethyl-D-erythro-sphingenine, synthetically prepared N,N-dimethyl-L-erythro-sphingenine, or pharmaceutically acceptable salts.

20. A method as claimed in claim 19 wherein the cell growth inhibitor comprises synthetically prepared N,N-dimethyl-D-erythro-sphingenine, or N,N-dimethylsphingosine prepared from naturally occurring sphingolipid or pharmaceutically acceptable salts.

21. A method as claimed in any one of claims 18, 19 or 20 wherein the cells are mammalian cells.

22. A method as claimed in any one of claims 18, 19 or 20 wherein the cells are malignant or benign tumor cells.

23. A medicament comprising:
i one or more cell growth inhibitors selected from synthetically prepared N-methyl sphingosine, N-methyl sphingosine prepared from naturally occuring sphingolipid and pharmaceutically acceptable salts thereof, and
ii a pharmaceutically acceptable carrier diluent or excipient.

24. A medicament as claimed in claim 23 wherein the cell growth inhibitor comprises N,N-dimethylsphingosine prepared from naturally occuring sphingolipid, N-monomethyl-sphingosine prepared from naturally occuring sphingolipid or synthetically prepared N,N-dimethyl-D-erythro-sphingenine, synthetically prepared N,N-dimethyl-L-erythro-sphingenine or pharmaceutically acceptable salts.

## Patentansprüche

1. Einer oder mehrere Zellwachstumsinhibitoren, ausgewählt aus synthetisch hergestelltem n-Methylsphingosin, N-Methylsphingosin, hergestellt aus natürlich vorkommendem Sphingolipid, und pharzmazeutisch annehmbaren Salzen zur Verwendung als Arzneimittel.

2. Zellwachstumsinhibitor gemäß Anspruch 1, umfassend N,N-Dimethylsphingosin, hergestellt aus natürlich vorkommendem Sphingolipid, oder N-Monomethylsphingosin, hergestellt aus natürlich vorkommendem Sphingolipid, oder synthetisch hergestelltes N-N-Dimethyl-D-erythrosphingenin, synthetisch hergestelltes N-N-Dimethyl-L-erythrosphingenin oder pharmazeutisch annehmbare Salze.

3. Zellwachstumsinhibitor gemäß Anspruch 2, umfassend synthetisch hergestelltes N,N-Dimethyl-D-erythrosphingenin oder N,N-Dimethylsphingosin, hergestellt aus natürlich vorkommendem Sphingolipid, oder pharmazeutisch annehmbare Salze.

4. Zellwachstumshinhibitor gemäß einem der Ansprüche 1 bis 3 zur Verwendung als Arzmeimittel für die Inhibierung von Säugerzellwachstum.

5. Zellwachstumsinhibitor gemäß einem der Ansprüche 1 bis 3 zur Verwendung als Arzneimittel bei der Behandlung bösartiger oder gutartiger Tumorzellen.

6. Verwendung von einem oder mehreren Zellwachstumsinhibitoren, ausgewählt aus synthetisch hergestelltem N-Methylsphingosin, N-Methylsphingosin, hergestellt aus natürlichem Sphingolipid, und pharmazeutisch annehmbaren Salzen davon, zur Herstellung eines Arzneimittels für die Inhibierung des Wachstums von Tumoren bei Menschen, Säugern oder anderen Tieren.

7. Verwendung von einem oder mehreren Zellwachstumsinhibitoren, ausgewählt aus synthetisch hergestelltem N-Methylsphinosin, N-Methylsphingosin, hergestellt aus natürlich vorkommendem Sphingolipid, und pharmazeutisch annehmbaren Salzen davon zur Herstellung eines Arzneimittels für die Inhibierung der Metastase von Tumoren bei Menschen, Säugern oder anderen Tieren.

8. Verwendung von einem oder mehreren Zellwachstumsinhibitoren, ausgewählt aus synthetisch hergestelltem N-Methylsphingosin, N-Methylsphingosin, hergestellt aus natürlich vorkommendem Sphingolipid, und pharmazeutisch annehmbaren Salzen davon zur Herstellung eines Arzneimittels für die Inhibierung von Immunreaktionen bei Mensch und Tier, vorzugsweise Säugern, mindestens teilweise beruhend auf von Lymphocyten-Mitogenese.

9. Verwendung gemäß Anspruch 8 zur Herstellung eines Arzneimittels für die Inhibierung einer Autoimmunantwort.

10. Verwendung gemäß Anspruch 9 zur Herstellung eines Arzneimittels für die Inhibierung einer Autoimmunantwort, mindestens teilweise beruhend auf dem Wachstum von IL-2-abhängigen T-Zellen.

11. Verwendung gemäß Anspruch 8 oder 9 zur Herstellung eines Arzneimittels für die Inhibierung einer Immunantwort, mindestens teilweise beruhend auf Conconavalin-A-induzierter Mitogenese von Lymphocyten.

12. Verwendung gemäß Anspruch 8 oder 9 zur Herstellung eines Arzneimittels für die Inhibierung einer Immunantwort, mindestens teilweise beruhend auf Phytohämagglutinin-A induzierter Mitogenese von Lymphocyten.

13. Verwendung von einem oder mehreren Wachstumsinhibitoren, ausgewählt aus synthetisch hergestelltem N-Methylsphingosin, N-Methylsphingosin, hergestellt aus natürlich vorkommendem Sphingolipid, und pharmazeutisch annehmbaren Salzen davon zur Herstellung eines Arzneimittels für die Inhibierung von menschlichen oder tierischen Entzündungsreaktionen, mindestens teilweise beruhend auf Granulocyten- oder Lymphocyten-Mitogenese.

14. Verwendung gemäß Anspruch 13 zur Herstellung eines Arzneimittel- für die Inhibierung von Entzündungsreaktionen bei Säugern.

15. Verwendung gemäß Anspruch 13, worin die Entzündungsreaktion zumindestens teilweise auf Conconavalin-A induzierte Mitogenese von Lymphocyten oder Phytohämagglutinin-A induzierte Mitogenese von Lymphocyten beruht.

16. Verwendung gemäß einem der Ansprüche 6 bis 15, worin die Zellwachstumsinhibitoren N,N-Dimethylsphingosin, hergestellt aus natürlich vorkommendem Sphingolipid, oder N-Monomethylsphingosin, hergestellt aus natürlich vorkommendem Sphingolipid oder synthetisch hergestelltes N-N-Dimethyl-D-erythrosphingenin, synthetisch hergestelltes N-N-Dimethyl-L-erythrosphingenin, oder pharmazeutisch annehmbare Salze umfassen.

17. Verwendung gemäß Anspruch 16, worin der Zellwachstumsinhibitor synthetisch hergestelltes N,N-Dimethyl-D-erythrosphingenin oder N,N-Dimethylsphingosin, hergestellt aus natürlichem Sphinoglipid, oder pharmazeutisch annehmbare Salze umfaßt.

18. Verfahren zur Inhibierung des Wachstums von humanen oder tierischen Zellen, das jedoch kein Verfahren zur Behandlung des menschlichen oder tierischen Körpers durch Eingriff oder Diagnose ist, das am menschlichen oder tierischen Körper durchgeführt wird, umfassend das In-Kontaktbringen der Zellen mit einem oder mehreren Zellwachstumsinhibitoren, ausgewählt aus synthetisch hergestelltem N-Methylsphingosin, N-Methylsphingosin, hergestellt aus natürlich vorkommendem Sphingolipid, und pharmazeutisch annehmbaren Salzen davon.

19. Verfahren gemäß Anspruch 18, worin der Zellwachstumsinhibitor N,N-Dimethylsphingosin, hergestellt aus natürlich vorkommendem Sphingolipid, oder N-46-Monomethylsphingosin, hergestellt aus natürlich vorkommendem Sphingolipid, oder synthetisch hergestelltes N,N-Dimethyl-D-erythrosphingenin, synthetisch hergestelltes N,N-Dimethyl-L-erythrosphingenin oder pharmazeutisch annehmbare Salze umfaßt.

20. Verfahren gemäß Anspruch 19, worin der Zellwachstumsinhibitor synthetisch hergestelltes N,N-Dimethyl-D-erythrosphingenin, oder N,N-Dimethylsphingosin, hergestellt aus natürlich vorkommendem Sphingolipid, oder pharmazeutisch annehmbare Salze umfaßt.

21. Verfahren gemäß einem der Ansprüche 18, 19 oder 20, worin die Zellen Säugerzellen sind.

22. Verfahren nach einem der Ansprüche 18, 19 oder 20, worin die Zellen bösartige oder gutartige Tumorzellen sind.

23. Arzneimittel, umfassend:
i einen oder mehrere Zellwachstumsinhibitoren, ausgewählt aus synthetisch hergestelltem N-Methylsphingosin, N-Methylsphingosin, hergestellt aus natürlich vorkommendem Sphingolipid, und pharmazeutisch annehmbare Salze davon, und
ii einen pharmazeutisch annehmbaren Träger, Verdünner oder Exzipienten.

24. Arzneimittel gemäß Anspruch 23, worin der Zellwachstumsinhibitor N,N-Dimethylsphingosin, hergestellt aus natürlich vorkommendem Sphingolipid, N-Monomethylsphingosin, hergestellt aus natürlich vorkommendem Sphingolipid oder synthetisch hergestelltes N,N-Dimethyl-D-erythrosphingenin, synthetisch hergestelltes N,N-Dimethyl-L-erythrosphingenin oder pharmazeutisch annehmbare Salze umfaßt.

## Revendications

1. Un ou plusieurs inhibiteurs de la croissance cellulaire choisis parmi la N-méthyl-sphingosine préparée synthétiquement, la N-méthyl-sphingosine préparée à partir de sphingolipide naturel et les sels pharmaceutiquement acceptables destinés à être utilisés comme médicament.

2. Inhibiteur de la croissance cellulaire selon la revendication 1 comprenant la N,N-diméthyl-sphingosine préparée à partir de sphingolipide naturel, ou la N-monométhyl-sphingosine préparée à partir de sphingolipide naturel ou de la N,N-diméthyl-D-érythro-sphingénine préparée synthétiquement, N,N-diméthyl-L-érythro-sphingénine préparée synthétiquement ou des sels pharmaceutiquement acceptables.

3. Inhibiteur de la croissance cellulaire selon la revendication 2 comprenant la N,N-diméthyl-D-érythro-sphingénine préparée synthétiquement ou la N,N-diméthyl-sphingosine préparée à partir de sphingolipide naturel, ou des sels pharmaceutiquement acceptables.

4. Inhibiteur de la croissance cellulaire selon l'une quelconque des revendications 1 à 3 pour l'utilisation dans un médicament destiné à inhiber la croissance cellulaire chez les mammifères.

5. Inhibiteur de la croissance cellulaire selon l'une quelconque des revendications 1 a 3 pour l'utilisation en tant que médicament destiné au traitement des cellules tumorales bénignes ou malignes.

6. Utilisation d'un ou plusieurs inhibiteurs de la croissance cellulaire choisis parmi la N-méthyl-sphingosine préparée synthétiquement, la N-méthyl-sphingosine préparée a partir de sphingolipide naturel et leurs sels pharmaceutiquement acceptables dans la fabrication d'un médicament destiné à inhiber la croissance de tumeurs chez l'homme, les mammifères ou d'autres animaux.

7. Utilisation d'un ou plusieurs inhibiteurs de la croissance cellulaire choisis parmi la N-méthyl-sphingosine préparée synthétiquement, la N-méthyl-sphingosine préparée à partir de sphingolipide naturel et leurs sels pharmaceutiquement acceptables dans la fabrication d'un médicament destiné à inhiber la métastase de tumeurs chez l'homme, les mammifères ou d'autres animaux.

8. Utilisation d'un ou plusieurs inhibiteurs de la croissance cellulaire choisis parmi la N-méthyl-sphingosine préparée synthétiquement, la N-méthyl-sphingosine préparée à partir de sphingolipide naturel et leurs sels pharmaceutiquement acceptables dans la fabrication d'un médicament destiné à inhiber les réponses immunitaires dues au moins en partie à la mitogénèse des lymphocytes chez l'homme et chez l'animal, de préférence chez les mammifères.

9. Utilisation selon la revendication 8 pour la fabrication d'un médicament destiné à inhiber une réponse auto-immunitaire.

10. Utilisation selon la revendication 9 pour la fabrication d'un médicament destiné à inhiber une réponse auto-immunitaire due au moins en partie à la croissance de cellules T dépendantes de la IL-2.

11. Utilisation selon la revendication 8 ou la revendication 9 pour la fabrication d'un médicament destiné à inhiber une réponse immunitaire due au moins en partie à la mitogénèse des lymphocytes induite par la conconavalline A .

12. Utilisation selon la revendication 8 ou la revendication 9 pour la fabrication d'un médicament destiné à inhiber une réponse immunitaire due au moins en partie à la mitogénèse des lymphocytes induite par la phyto-hémagglutinine A.

13. Utilisation d'un ou plusieurs inhibiteurs de la croissance cellulaire choisis parmi la N-méthyl-sphingosine préparée synthétiquement, la N-méthyl-sphingosine préparée à partir de sphingolipide naturel et leurs sels pharmaceutiquement acceptables pour la fabrication d'un médicament destiné à inhiber, chez l'homme et chez l'animal, une réponse inflammatoire due au moins en partie à la mitogénèse des granulocytes ou des lymphocytes.

14. Utilisation selon la revendication 13 pour la fabrication d'un médicament destiné à inhiber la réponse inflammatoire chez les mammifères.

15. Utilisation selon la revendication 13, dans laquelle la réponse inflammatoire est due au moins en partie à la mitogénèse des lymphocytes induite par la conconavalline A ou à la mitogénèse des lymphocytes induite par la phyto-hémagglutinine A.

16. Utilisation selon l'une quelconque des revendications 6 à 15, dans laquelle les inhibiteurs de la croissance cellulaire comprennent la N,N-diméthyl-sphingosine préparée à partir de sphingolipide naturel, ou la N-monométhyl-sphingosine préparée à partir de sphingolipide naturel ou de la N,N-diméthyl-D-érythro-sphingénine préparée synthétiquement, N,N-diméthyl-L-érythro-sphingénine préparée synthétiquement, ou des sels pharmaceutiquement acceptables.

17. Utilisation selon la revendication 16, dans laquelle l'inhibiteur de la croissance cellulaire comprend la N,N-diméthyl-D-érythro-sphingénine préparée synthétiquement ou la N,N-diméthyl-sphingosine préparée à partir de sphingolipide naturel, ou des sels pharmaceutiquement acceptables.

18. Procédé d'inhibition de la croissance de cellules humaines ou animales, autre qu'un procédé destiné au traitement de l'organisme humain ou animal par traitement chirurgical ou au diagnostic réalisé sur l'organisme humain ou animal, comprenant la mise en contact de cellules avec un ou plusieurs inhibiteurs de la croissance cellulaire choisis parmi la N-méthyl-sphingosine préparée synthétiquement, la N-méthyl-sphingosine préparée à partir de sphingolipide naturel et leurs sels pharmaceutiquement acceptables.

19. Méthode selon la revendication 18, dans laquelle l'inhibiteur de la croissance cellulaire comprend la N,N-diméthyl-sphingosine préparée à partir de sphingolipide naturel, ou la N-46-monométhyl-sphingosine préparée à partir de sphingolipide naturel ou de la N,N-diméthyl-D-érythro-sphingénine préparée synthétiquement, N,N-diméthyl-L-érythro-sphingénine préparée synthétiquement, ou des sels pharmaceutiquement acceptables.

20. Méthode selon la revendication 19, dans laquelle l'inhibiteur de la croissance cellulaire comprend la N,N-diméthyl-D-érythro-sphingénine préparée synthétiquement, la N,N-diméthyl-sphingosine préparée à partir de sphingolipide naturel ou des sels pharmaceutiquement acceptables.

21. Méthode selon l'une quelconque des revendications 18, 19 ou 20, dans laquelle les cellules sont des cellules de mammifères.

22. Méthode selon l'une quelconque des revendications 18, 19 ou 20, dans laquelle les cellules sont des cellules tumorales bénignes ou malignes.

23. Médicament comprenant :
(i) un ou plusieurs inhibiteurs de la croissance cellulaire choisis parmi la N-méthyl-sphingosine préparée synthétiquement, la N-méthyl-sphingosine préparée à partir de sphingolipide nature et leurs sels pharmaceutiquement acceptables, et
(ii) un diluant ou excipient vecteur, pharmaceutiquement acceptable.

24. Médicament selon la revendication 23, dans lequel l'inhibiteur de la croissance cellulaire comprend la N,N-diméthyl-sphingosine préparée à partir de sphingolipide naturel, la N-monométhyl-sphingosine préparée à partir de sphingolipide naturel ou de la N,N-diméthyl-D-érythro-sphingénine, N,N-diméthyl-L-érythro-sphingénine préparée synthétiquement, ou les sels pharmaceutiquement acceptables.
